# EUROPEAN PATENT SPECIFICATION

(11) **EP 3 766 496 B1**
(45) Date of publication and mention of the grant of the patent: **25.02.2026**
(21) Application number: 19768631.4
(22) Date of filing: 07.03.2019
(51) Int. Cl.: A61K 31/4709, A61P 35/00

(54) **ANLOTINIB FOR TREATMENT OF NASOPHARYNGEAL CARCINOMA**
ANLOTINIB ZUR BEHANDLUNG DES NASOPHARYNGEALEN KARZINOMS
ANLOTINIB POUR LE TRAITEMENT DU CARCINOME DU NASOPHARYNX

(30) Priority: 14.03.2018 CN 201810206825
(43) Date of publication of application: 20.01.2021
(73) Proprietor: Chia Tai Tianqing Pharmaceutical Group Co., Ltd., Lianyungang, Jiangsu 222062 (CN)
(72) Inventor: WANG, Shanchun, Lianyungang, Jiangsu 222062 (CN); SHEN, Jun, Lianyungang, Jiangsu 222062 (CN); HAN, Xi, Lianyungang, Jiangsu 222062 (CN); WANG, Xunqiang, Lianyungang, Jiangsu 222062 (CN); TANG, Shaonan, Lianyungang, Jiangsu 222062 (CN); CAO, Shuqing, Lianyungang, Jiangsu 222062 (CN); ZHANG, Xiquan, Lianyungang, Jiangsu 222062 (CN); YU, Hao, Lianyungang, Jiangsu 222062 (CN); PAN, Maoqiong, Lianyungang, Jiangsu 222062 (CN)
(74) Representative: v. Bezold & Partner Patentanwälte - PartG mbB
(86) International application number: PCT/CN2019/077244
(87) International publication number: WO 2019/174508

(56) References cited:
- WO-A1-2008/112407
- WO-A1-2017/118401
- WO-A1-2020/057536
- CN-A- 105 311 030
- PENG QIU-XIA ET AL: "Apatinib inhibits VEGFR-2 and angiogenesis in an in vivo murine model of nasopharyngeal carcinoma", ONCOTARGET, vol. 8, no. 32, 8 August 2017 (2017-08-08), pages 52813 - 52822, XP055860440, DOI: 10.18632/oncotarget.17264
- PERRI F ET AL: "Management of recurrent nasopharyngeal carcinoma: current perspectives", ONCOTARGETS AND THERAPY, vol. Volume 12, 1 January 2019 (2019-01-01), pages 1583 - 1591, XP093207727, ISSN: 1178-6930, Retrieved from the Internet <URL:https://www.dovepress.com/article/download/44324> DOI: 10.2147/OTT.S188148
- HO CS ET AL: "Beating 'Guangdong cancer': a review and update on nasopharyngeal cancer", HONG KONG MEDICAL JOURNAL, 1 October 2017 (2017-10-01), HK, XP093207724, ISSN: 1024-2708, Retrieved from the Internet <URL:https://www.hkmj.org/system/files/hkmj176834.pdf> DOI: 10.12809/hkmj176834

## Description

### FIELD OF THE INVENTION

The present invention belongs to the technical field of medicine, and specifically relates to use of a quinoline derivative for use in preventing and/or treating nasopharyngeal carcinoma.

### BACKGROUND OF THE INVENTION

Nasopharyngeal carcinoma is a malignant tumor that occurs in the top and side walls of the nasopharyngeal cavity, which is one of the most frequent malignant tumors in China with the highest incidence in otolaryngological malignant tumors. The World Health Organization (WHO) classifies nasopharyngeal carcinoma into two types: keratinizing squamous cell carcinoma and non-keratinizing carcinoma (including differentiated and undifferentiated), and the most important difference between the two types is whether there is an obvious evidence for keratinization. The former has an obvious keratinization, and is more common in the elderly and not closely related to EB virus infection. The latter accounts for the majority of nasopharyngeal carcinoma and has no obvious keratosis, and they, especially undifferentiated type, are closely related to EB virus infection. In non-keratinizing carcinoma, differentiated carcinoma cells have clear boundaries, arranged in multiple layers or pavements; while undifferentiated carcinoma cells have unclear boundaries and are in a form of syncytialization, and some are spindle-shaped. Abundant lymphocyte infiltration is commonly found in non-keratinizing carcinoma, especially undifferentiated type. In China, nasopharyngeal carcinoma is often divided into two classes: carcinoma in situ and infiltrating carcinoma. Infiltrating carcinoma includes 5 subtypes: microinvasive carcinoma, squamous cell carcinoma (with high, medium, and low differentiation), adenocarcinoma (with high, medium and low differentiation), vesicular nucleus cell carcinoma, and undifferentiated carcinoma.

Peng Qiu-Xia et al., Oncotarget, vol. 8, no. 32, 8. August 2017, pages 52813-52822, disclose that Apatinib inhibits VEGFR-2 and angiogenesis in an in vivo murine model of nasopharyngeal carcinoma.

Size, Chen, et al. pointed out in the article of "Diagnostic Significance and Function Study of NLK Expression in Nasopharyngeal Carcinoma" that most of nasopharyngeal carcinomas are moderately sensitive to radiotherapy, and thus radiotherapy is the first choice for the treatment of nasopharyngeal carcinoma. However, for the cases with highly differentiated carcinoma, later course of the disease and recurrence after radiotherapy, surgical resection and chemotherapy are also indispensable means. Since the complicated structure of nasopharynx and the lesions are deep and hidden, the difficulty of treatment is increased. Meanwhile, there is a lack of individualized specific treatment methods and drugs, and the therapies are relatively simple. Therefore, the treatment effect on nasopharyngeal carcinoma is still poor, and the 5-year survival rate remains low.

### SUMMARY OF THE INVENTION

In an aspect, the present invention provides a compound of Formula (I) or a pharmaceutically acceptable salt thereof for use in preventing and/or treating nasopharyngeal carcinoma, wherein the nasopharyngeal carcinoma is advanced and/or metastatic and/or recurrent nasopharyngeal carcinoma.

The chemical name of the compound of Formula (I) is 1-[[[4-(4-fluoro-2-methyl-1H-indol-5-yl)oxy-6-methoxyquinolin-7-yl]oxy]methyl]cyclopropylamine, and it has the following structural formula:

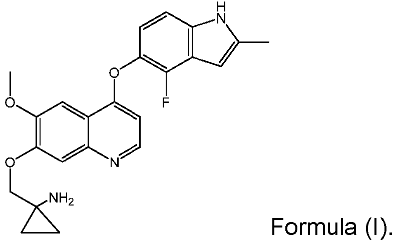

In some particular embodiments of the present invention, the nasopharyngeal carcinoma is keratinizing squamous cell carcinoma or non-keratinizing nasopharyngeal carcinoma; preferably the non-keratinizing nasopharyngeal carcinoma is differentiated or undifferentiated type.

In some particular embodiments of the present invention, the nasopharyngeal carcinoma is nasopharyngeal carcinoma in situ or infiltrating nasopharyngeal carcinoma; preferably the infiltrating nasopharyngeal carcinoma is squamous cell carcinoma, adenocarcinoma, microinvasive carcinoma, vesicular nucleus cell carcinoma or undifferentiated nasopharyngeal carcinoma.

In some particular embodiments of the present invention, the advanced and/or metastatic nasopharyngeal carcinoma metastasizes to the neck lymph, spleen and/or lung. In some particular embodiments of the present invention, the nasopharyngeal carcinoma cannot be surgically removed. In some particular embodiments of the present invention, a patient with nasopharyngeal carcinoma has previously received chemotherapy, monoclonal antibody treatment, and/or radiotherapy. In some preferred embodiments of the present invention, the disease progresses after the patient with nasopharyngeal carcinoma previously receiving chemotherapy, monoclonal antibody treatment, and/or radiotherapy. In some more preferred embodiments of the present invention, the chemotherapeutic agents previously received by the patient with nasopharyngeal carcinoma include gemcitabine, capecitabine, cisplatin, lobaplatin, nedaplatin, 5-fluorouracil, paclitaxel, docetaxel and/or cyclophosphamide; the monoclonal antibody therapeutic agents previously received by the patient with nasopharyngeal carcinoma include cetuximab, bevacizumab, and/or SHR-1210.

Compound (I) can be administered in the free base form thereof, and can also be administered in the form of a salt, hydrate and prodrug thereof (the prodrug will be converted into the free base form of Compound (I) in the body). For example, the pharmaceutically acceptable salt of Compound (I) is within the scope of the present invention, and the salt can be produced by different organic acids and inorganic acids in accordance with well-known processes in the art.

In some particular embodiments of the present invention, Compound (I) is administered in a form of hydrochloride thereof. In some particular embodiments, Compound (I) is administered in a form of monohydrochloride or dihydrochloride thereof. In some particular embodiments, Compound (I) is administered in a crystalline form of hydrochloride thereof. In some particular embodiments, Compound (I) is administered in the crystalline form of dihydrochloride thereof.

The compound of Formula (I) or the pharmaceutically acceptable salt thereof can be administered via various routes, including but not limited to: orally, parenterally, intraperitoneally, intravenously, intraarterially, transdermally, sublingually, intramuscularly, rectally, transbuccally, intranasally, via inhalation, vaginally, intraocularly, via local administration, subcutaneously, intraadiposally, intraarticularly, and intrathecally. In some particular embodiments, the administration is performed orally, and the specific formulations include tablets, capsules, dusts, granulates, drip pills, pastes, powders and the like, and tablets and capsules are preferred. Among them, the tablets can be common tablets, dispersible tablets, effervescent tablets, sustained release tablets, controlled release tablets or enteric coated tablets, and the capsules can be common capsules, sustained release capsules, controlled release capsules or enteric coated capsules. The oral formulations can be prepared with well-known pharmaceutically acceptable carriers in the art by conventional methods. The pharmaceutically acceptable carriers include bulking agents, absorbing agents, wetting agents, binding agents, disintegrating agents, lubricants and the like. The bulking agents include starch, lactose, mannitol, microcrystalline cellulose and the like; the absorbing agents include calcium sulfate, calcium hydrogen phosphate, calcium carbonate and the like; the wetting agents include water, ethanol and the like; the binding agents include hydroxypropyl methylcellulose, povidone, microcrystalline cellulose and the like; the disintegrating agents include cross-linked carboxymethyl cellulose sodium, crospovidone, surfactants, low-substituted hydroxypropyl cellulose and the like; the lubricants include magnesium stearate, talc powder, polyethylene glycol, sodium dodecylsulfate, Aerosil, talc powder and the like. The pharmaceutical excipients also include colorants, sweetening agents and the like.

In some particular embodiments of the present invention, the daily administration dosage to the patient can be 2 mg to 20 mg; in some particular embodiments, the daily administration dosage to the patient is 5 mg to 20 mg; in some particular embodiments, the daily administration dosage to the patient is 10 mg to 16 mg; in some particular embodiments of the present invention, the daily administration dosage to the patient is 10 mg to 14 mg; in some particular embodiments, the daily administration dosage to the patient is 8 mg, 10 mg, 12 mg, 14 mg or 16 mg.

The compound of Formula (I) or a pharmaceutically acceptable salt thereof can be administered one or more times daily in a unit dose or multiple doses. In some particular embodiments of the present invention, the compound of Formula (I) or a pharmaceutically acceptable salt thereof is administered once per day.

The administration amount of the compound of Formula (I) or a pharmaceutically acceptable salt thereof can be determined according to severity of diseases, the response of diseases, any treatment-related toxicity, and age and health status of patients. Preferably, the compound of Formula (I) or a pharmaceutically acceptable salt thereof is administered in the manner of interval administration. The interval administration includes administration periods and rest periods, and during the administration periods, the compound of Formula (I) or a pharmaceutically acceptable salt thereof can be administered one or more times daily. For example, the compound of Formula (I) or a pharmaceutically acceptable salt thereof is administered daily in an administration period, and then the administration is stopped for a period of time in a rest period, followed by an administration period and then a rest period, such an administration regimen can be repeated many times. Among them, the ratio of the administration period to the rest period in days is 2:0.5~5, preferably 2:0.5~3, more preferably 2:0.5~2, most preferably 2:0.5-1.

In some particular embodiments, the administration is continuously performed for 2 weeks and rest for 2 weeks. In some particular embodiments, the administration is continuously performed once daily for 14 days and rest for 14 days, followed by continuously administering once daily for 14 days and resting for 14 days, such an interval administration regimen with a 14-day continuous administration period and a 14-day rest period can be repeated many times.

In some particular embodiments, the administration is continuously performed for 2 weeks and rest for 1 week. In some particular embodiments, the administration is continuously performed once daily for 14 days and rest for 7 days, followed by continuously administering once daily for 14 days and resting for 7 days, such an interval administration regimen with a 14-day continuous administration period and a 7-day rest period can be repeated many times.

In some particular embodiments, the administration is continuously performed for 5 days and rest for 2 days. In some particular embodiments, the administration is continuously performed once daily for 5 days and rest for 2 days, followed by continuously administering once daily for 5 days and resting for 2 days, such an interval administration regimen with a 5-day continuous administration period and a 2-day rest period can be repeated many times.

In some embodiments, the compound of Formula (I) or a pharmaceutically acceptable salt thereof is administered to the patient with nasopharyngeal carcinoma alone as the sole active ingredient. In some embodiments, the compound of Formula (I) or a pharmaceutically acceptable salt thereof is administered with other antitumor drugs simultaneously or sequentially to the patient with nasopharyngeal carcinoma. In some embodiments, other antitumor drugs include but not limited to an alkylating agent, a platinum complex, a fluoropyrimidine derivative, camptothecin and a derivative thereof, an antitumor antibiotic of anthraquinones, a taxanes compound or a monoclonal antibody anticancer drug.

In another aspect, the present invention provides a pharmaceutical composition for use in treating nasopharyngeal carcinoma comprising the compound of Formula (I) or a pharmaceutically acceptable salt thereof, and at least one pharmaceutically acceptable carrier.

In yet another aspect, the present invention provides a kit for use in treating nasopharyngeal carcinoma, comprising (a) at least one unit dose of a pharmaceutical composition of the compound of Formula (I) or a pharmaceutically acceptable salt thereof, and (b) instructions for treatment of nasopharyngeal carcinoma.

Unless indicated otherwise, for the purpose of the present application, the following terms used in the description and claims are intended to have the meanings denoted below. A "patient" refers to mammal, preferably human.

"Pharmaceutically acceptable" means those which are useful in preparing a pharmaceutical composition that is generally safe, non-toxic and neither biologically nor otherwise undesirable, and that is acceptable for human pharmaceutical use when the carriers are included.

"Pharmaceutically acceptable salt" includes, but not limited to acid addition salts formed from inorganic acids, such as hydrochloric acid, hydrobromic acid, sulfuric acid, nitric acid, phosphoric acid and the like; or acid addition salts formed from organic acids, such as acetic acid, trifluoroacetic acid, propionic acid, caproic acid, heptanoic acid, cyclopentane propionic acid, glycolic acid, pyruvic acid, lactic acid, malonic acid, succinic acid, malic acid, maleic acid, fumaric acid, tartaric acid, citric acid, benzoic acid, cinnamic acid, mandelic acid, methyl sulfonic acid, ethyl sulfonic acid, 1,2-ethanedisulfonic acid, 2-hydroxyethanesulphonic acid, benzenesulfonic acid, p-chlorobenzenesulfonic acid, p-toluenesulfonic acid, 3-phenylpropionic acid, trimethylacetic acid, t-butylacetic acid, dodecyl sulfuric acid, gluconic acid, glutamic acid, hydroxynaphthoic acid, salicylic acid, stearic acid and the like.

"Therapeutically effective amount" means an amount of a compound that, when administered to human for treating a disease, is sufficient to achieve control of the disease.

"Treatment/treating" means any administration of a therapeutically effective amount of a compound, and includes:
(1) Inhibiting a disease in a human body that is experiencing or displaying the pathology or symptomatology of the disease (i.e., retarding further progression of the pathology and/or symptomatology), or
(2) Ameliorating a disease in a human body that is experiencing or displaying the pathology or symptomatology of the disease (i.e., reversing the pathology and/or symptomatology).

"CR" refers to complete remission, and specifically means that when target lesions of tumors disappear, no new lesions appear and the tumor markers are normal, which is maintained for at least 4 weeks.

"PR" refers to partial remission, and specifically means 30% or more decrease in the sum of the diameters of target lesions of tumors than the baseline level, which is maintained for at least 4 weeks.

"PD" refers to the progression of disease, and specifically means 20% or more increase in the diameters of target lesions of tumors than the baseline level.

"SD" refers to stable disease, and specifically means that the decrease degree of target lesions of tumors does not reach the PR level, and the increase degree does not reach the PD level either, falling somewhere therebetween.

"qd" refers to taking the drug once per day.

"Advanced" includes "locally advanced".

### DETAILED EMBODIMENTS OF THE INVENTION

### Example 1 1-[[[4-(4-fluoro-2-methyl-1H-indol-5-yl)oxy-6-methoxyquinolin-7-yl]oxy]methyl]cyclopropylamine dihydrochloride

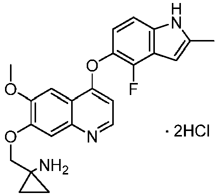

1-[[[4-(4-fluoro-2-methyl-1H-indol-5-yl)oxy-6-methoxyquinolin-7-yl]oxy]methyl]cyclopropylamine was prepared by reference to the method of Example 24 in WO2008112407, and then the title compound was prepared by reference to the preparation method in "Examples of Salt Formation" of the Description of WO2008112407.

### Example 2 Preparation of capsules of 1-[[[4-(4-fluoro-2-methyl-1H-indol-5-yl)oxy-6-methoxyquinolin-7-yl]oxy]methyl]cyclopropylamine dihydrochloride (the compound of Example 1)

| Active ingredient/excipient | amount (1000 capsules) |
|---|---|
| The compound of Example 1 | 14.16 g |
| Mannitol | 89 g |
| Microcrystalline cellulose | 138.4 g |
| Hydroxypropyl cellulose | 5.9 g |
| Magnesium stearate | 0.99 g |

The compound of Example 1 was grinded and sifted with a 80 mesh sieve, and then mixed uniformly with mannitol and hydroxypropyl cellulose; the prescribed amount of microcrystalline cellulose was subsequently added, mixed uniformly and sifted with a 0.8mm sieve; and finally, the prescribed amount of magnesium stearate was added and mixed uniformly, and the obtained mixture was filled into capsules.

The capsule in which dihydrochloride of Compound I is at different content can be prepared by reference to the above proportion and formulation.

### Example 3

Screening of enrolled patients:
1) Having been definitely diagnosed with nasopharyngeal carcinoma based on pathology or cytology;
2) With measurable lesions (according to RECIST 1.1 standard);
3) Lack of effective conventional treatment methods, or conventional treatment failure or relapse;
4) The body mass index (BMI) meets: 20≤BMI≤25;
5) 18≤age≤70; ECOG score: 0 to 1; expected survival time: more than 3 months;
6) The function of the main organs is normal;
7) Women should agree to take contraceptive measures (e.g. intrauterine device [IUD], condom) during the study period and within 6 months after the end of the study period, should be negative for serum or urine pregnancy test within 7 days before enrolled to the study, and must be non-lactating subjects; and men should agree to take contraceptive measures during the study period and within 6 months after the end of the study period;
8) The subjects voluntarily join the study, sign an informed consent form, and have good compliance.

### Treatment solutions:

During or after radiotherapy, the patients are treated with the capsules of the compound of Example 1 at 12 mg qd. The administration is continuously performed for 2 weeks and rest for 1 week, followed by continuously administering for 2 weeks and resting for 1 week as a cycle of continuous administration. The target lesions are regularly evaluated, and the treatment regimen is ended according to the evaluation results.

### Example 4 Efficacy on nasopharyngeal carcinoma

A 53-year-old male patient was diagnosed with nasopharyngeal non-keratinizing undifferentiated carcinoma by nasopharyngoscopy biopsy on November 19, 2014. On November 29, 2014, MRI on nasopharynx indicated multiple swollen lymph nodes in the bilateral jaws and lower neck.

From December 16, 2014 to January 26, 2015, paclitaxel + cisplatin+5-fluorouracil were administered to induce chemotherapy for 3 cycles, and the chemotherapy was successful. From February 25, 2015 to April 2015, 68 Gy was given to the nasopharynx and 70 Gy to the left and right neck lymph nodes for radiotherapy, with a total of 30 times; and treatment of cetuximab + cisplatin chemotherapy was performed at the same period. Subsequent periodic reexamination showed the disease was stable.

On November 20, 2015, PET/CT showed spleen metastasis after nasopharyngeal carcinoma radiotherapy, indicating progression of the disease. Based on the EBV-DNA results on January 8, 2016, it was considered a recurrence of nasopharyngeal carcinoma. From January 27, 2016 to May 27, 2016, the patient was enrolled in the ML29153 clinical trial with informed consent, and treated with docetaxel + cyclophosphamide + bevacizumab chemotherapy for 6 cycles, the treatment was completed successfully. Adverse reactions such as bone marrow suppression occurred during the treatment, which was relieved after symptomatic treatment.

From June 2016 to July 2017, bevacizumab was administered for 14 cycles, and the tumors were stable by re-examination during the administration. Afterwards, re-examination indicated that multiple lymph nodes appeared adjacent the abdominal aorta, evaluated as PD.

Subsequently, the patient took orally the capsules of 12 mg of the compound of Example 1 once per day for treatment (continuously administering for 2 weeks and resting for 1 week as a treatment cycle). After 2 cycles of the treatment, the enhanced CT showed that the left anterior lymph nodes of the abdominal aorta and the right anterior lymph nodes of the abdominal aorta were slightly smaller than before, evaluated as PR according to RECIST 1.1; and and the sum of the target lesions was 12 mm, which was reduced by 7 mm from the baseline. After 4 cycles of treatment, he was evaluated as PR according to RECIST 1.1, and the sum of the target lesions was 10 mm, which was reduced by 9 mm from the baseline. After 8 cycles of treatment, the sum of the target lesions was 9 mm, which was reduced by 10 mm from the baseline.

Grade 3 hypertension occurred during the administration of the capsules of the compound of Example 1, and the symptoms of hypertension were relieved after measures of medication and dosage reduction.

### Example 5 Efficacy on nasopharyngeal carcinoma

A 58-year-old female patient was suspected of nasopharyngeal carcinoma through PET-CT in February 2012, and was diagnosed as non-keratinizing undifferentiated carcinoma by biopsy pathology. CT showed metastasis in the right retropharyngeal lymph nodes, the lymph nodes in level II of the right neck and the nodules in the right middle lung.

Gemcitabine + cisplatin chemotherapy was given from April 18, 2012 to August 22, 2012 for 6 cycles, and the best overall response was PR. On April 21, 2016, CT showed enlarged lung masses and axillary lymph nodes when reexamination, indicating disease progression.

From June 16, 2016 to October 18, 2016, docetaxel + cyclophosphamide chemotherapy was given for 6 cycles, and the best overall response was PR. Grade 3 leukopenia, Grade 2 platelet decrease and symptoms of low fever occurred during treatment, which was alleviated after appropriate treatment. CT for re-examnation on February 10, 2017 indicated disease progression.

From March 2, 2017, the patient started to take orally the capsules of 12 mg of the compound of Example 1 once per day for treatment (continuously administering for 2 weeks and resting for 1 week as a treatment cycle). On April 12, 2017, after 2 cycles of treatment, the enhanced CT showed that the nodules in the middle lobe of the right lung were smaller than before, evaluated as PR according to RECIST 1.1; and the sum of the target lesions was 30 mm, which was reduced by 17 mm from the baseline. The treatment with the capsules of the compound of Example 1 was continued.

On May 22, 2017, after 4 cycles of the chemotherapy, the enhanced CT showed that the nodules in the middle lobe of the right lung was slightly smaller than before, evaluated as PR according to RECIST 1.1; and the sum of the target lesions was 28 mm, which was reduced by 19 mm from the baseline. The treatment with the capsules of the compound of Example 1 was continued. On July 3, 2017, after 6 cycles of the treatment, the enhanced CT showed that the nodules in the middle lobe of the right lung was slightly smaller than before, evaluated as PR according to RECIST 1.1; and the sum of the target lesions was 24 mm, which was reduced by 23 mm from the baseline. The treatment with the capsules of the compound of Example 1 was continued. From August 14, 2017 to November 6, 2017, after 12 cycles of the treatment, the enhanced CT showed no change in the nodules in the middle lobe of the right lung from before, evaluated as PR according to RECIST 1.1; and the results of the efficacy evaluation showed that the sum of the target lesions was 24 mm, which was reduced by 23 mm from the baseline. On January 29, 2018, after 16 cycles of the chemotherapy, the enhanced CT showed no change in the nodules in the middle lobe of the right lung from before, evaluated as PR according to RECIST 1.1; and the results of the efficacy evaluation showed that the sum of the target lesions was 23 mm, which was reduced by 24 mm from the baseline.

### Example 6 Efficacy on nasopharyngeal carcinoma

A 44-year-old male patient was diagnosed with undifferentiated nasopharyngeal non-keratinizing carcinoma by nasopharyngoscopy biopsy. MRI on the nasopharynx and neck indicated multiple swollen lymph nodes and metastasis in the right carotid artery sheath and deep neck.

Subsequently, a combined chemotherapy with docetaxel + cisplatin + capecitabine was administered for 3 cycles, and the best overall response reached PR, with mild adverse reactions during this period. IMRT precise radiotherapy was performed 1 month after the end of the chemotherapy, with the doses of GTV 70Gy/33f, GTVnd(R/L) 66Gy/33f, and CTV 62Gy/33f; and cisplatin was administered at the same period. The condition was stable after 2 cycles of the chemotherapy, and then periodic reexamination was performed.

CT for reexamination showed multiple nodules dispersing in both lungs, indicating the progression of the disease. The pathological results of the lung masses by puncture biopsy confirmed the diagnosis of Stage IV of pulmonary metastasis after radiotherapy and chemotherapy of nasopharyngeal non-keratinizing undifferentiated carcinoma. A combined chemotherapy with Gemcitabine + cisplatin + SHR-1210 was administered for 6 cycles, and the best overall response reached PR, with occurrence of Grade 2 leukopenia after treatment and then improvement after treatment of increasing leucocyte. A combined chemotherapy with Gemcitabine + cisplatin + SHR-1210 was then performed continually for 8 cycles, and the best overall response reached PR, and afterwards he was evaluated as the progression of the disease (PD).

3 months later, the patient started to take orally the capsules of 12 mg of the compound of Example 1 once per day for treatment (continuously administering for 2 weeks and resting for 1 week as a treatment cycle). After 2 cycles of the treatment, the enhanced CT showed that the lymph nodes next the right lower bronchus, the nodules in the right lower lung, and the nodules in the right adrenal gland were slightly smaller than before, evaluated as SD according to RECIST 1.1; and the sum of the target lesions was 47 mm, which was reduced by 18 mm from the baseline. The treatment with the capsules of the compound of Example 1 was continued. After 4 cycles of the treatment, the enhanced CT showed that the lymph nodes next the right lower bronchus, the nodules in the right lower lung, and the nodules in the right adrenal gland were slightly smaller than before, evaluated as PR according to RECIST 1.1; and the sum of the target lesions was 42 mm, which was reduced by 23 mm from the baseline.

Grades 2 and 3 hypertension occurred successively during the administration of the capsules of the compound of Example 1, and then were alleviated by administration of antihypertensive drugs at the same period, and reducing the dose to 10 mg and 8 mg successively.

## Claims

1. A compound of Formula (I) or a pharmaceutically acceptable salt thereof for use in preventing and/or treating nasopharyngeal carcinoma, wherein the nasopharyngeal carcinoma is advanced and/or metastatic and/or recurrent nasopharyngeal carcinoma,

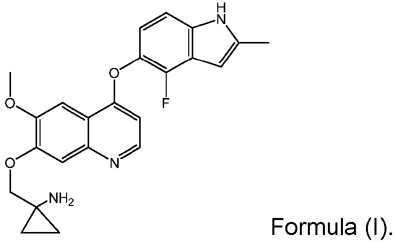

2. The compound or the pharmaceutically acceptable salt thereof for use of claim 1, the nasopharyngeal carcinoma is keratinizing squamous cell carcinoma or non-keratinizing nasopharyngeal carcinoma; preferably, the non-keratinizing nasopharyngeal carcinoma is differentiated or undifferentiated type.

3. The compound or the pharmaceutically acceptable salt thereof for use of claim 1, wherein the nasopharyngeal carcinoma is nasopharyngeal carcinoma in situ or infiltrating nasopharyngeal carcinoma; preferably, the infiltrating nasopharyngeal carcinoma is squamous cell carcinoma, adenocarcinoma, microinvasive carcinoma, vesicular nucleus cell carcinoma or undifferentiated nasopharyngeal carcinoma.

4. The compound or the pharmaceutically acceptable salt thereof for use of claim 1, wherein the advanced and/or metastatic nasopharyngeal carcinoma metastasizes to neck lymph, spleen and/or lung.

5. The compound or the pharmaceutically acceptable salt thereof for use of claim 1, wherein the nasopharyngeal carcinoma cannot be surgically removed.

6. The compound or the pharmaceutically acceptable salt thereof for use of claim 1, wherein a patient with nasopharyngeal carcinoma has previously received chemotherapy, monoclonal antibody treatment, and/or radiotherapy; preferably, the patient with nasopharyngeal carcinoma has disease progression after previously receiving chemotherapy, monoclonal antibody treatment, and/or radiotherapy.

7. The compound or the pharmaceutically acceptable salt thereof for use of claim 6, wherein the chemotherapeutic agent previously received by the patient with nasopharyngeal carcinoma includes gemcitabine, capecitabine, cisplatin, lobaplatin, nedaplatin, 5-fluorouracil, paclitaxel, docetaxel, and/or cyclophosphamide; the monoclonal antibody therapeutic agent previously received by the patient with nasopharyngeal carcinoma includes cetuximab, bevacizumab, and/or SHR-1210.

8. The compound or the pharmaceutically acceptable salt thereof for use of any one of claims 1 to 7, wherein the pharmaceutically acceptable salt of the compound of Formula (I) is a salt formed by the compound of Formula (I) with any of the following acids: hydrochloric acid, hydrobromic acid, sulfuric acid, nitric acid, phosphoric acid, acetic acid, trifluoroacetic acid, propionic acid, caproic acid, heptanoic acid, cyclopentane propionic acid, glycolic acid, pyruvic acid, lactic acid, malonic acid, succinic acid, malic acid, maleic acid, fumaric acid, tartaric acid, citric acid, benzoic acid, cinnamic acid, mandelic acid, methyl sulfonic acid, ethyl sulfonic acid, 1,2-ethanedisulfonic acid, 2-hydroxyethanesulphonic acid, benzenesulfonic acid, p-chlorobenzenesulfonic acid, p-toluenesulfonic acid, 3-phenylpropionic acid, trimethylacetic acid, t-butylacetic acid, dodecyl sulfuric acid, gluconic acid, glutamic acid, hydroxynaphthoic acid, salicylic acid, stearic acid; preferably is hydrochloride, and more preferably dihydrochloride.

9. The compound or the pharmaceutically acceptable salt thereof for use of any one of claims 1 to 7, wherein a daily administration dosage is 2 mg to 20 mg, preferably 5 mg to 20 mg, more preferably 10 mg to 16 mg, more preferably 10 mg to 14 mg, more preferably 8 mg, 10 mg, 12 mg, 14 mg or 16 mg.

10. The compound or the pharmaceutically acceptable salt thereof for use of any one of claims 1 to 7, wherein the compound of Formula (I) or the pharmaceutically acceptable salt thereof is administered by an interval administration regimen including administration periods and rest periods, and wherein a ratio of the administration period to the rest period in days is 2 : 0.5-5, preferably 2 : 0.5-3, more preferably 2 : 0.5-2, and more preferably 2 : 0.5-1.

11. The compound or the pharmaceutically acceptable salt thereof for use of claim 10, wherein the interval administration regimen including administration periods and rest periods is continuously administering for 2 weeks and resting for 2 weeks, or continuously administering for 2 weeks and resting for 1 week, or continuously administering for 5 days and resting for 2 days.

12. A pharmaceutical composition for use in treating nasopharyngeal carcinoma, comprising a compound of Formula (I) or a pharmaceutically acceptable salt thereof, and at least one pharmaceutically acceptable carrier

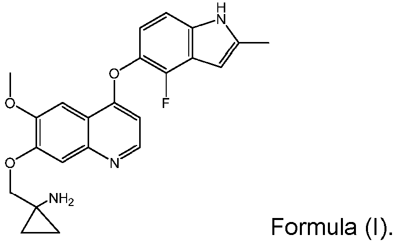

13. A kit for use in treating nasopharyngeal carcinoma, comprising (a) at least one unit dose of a pharmaceutical composition of a compound of Formula (I) or a pharmaceutically acceptable salt thereof, and (b) instructions for treatment of nasopharyngeal carcinoma,

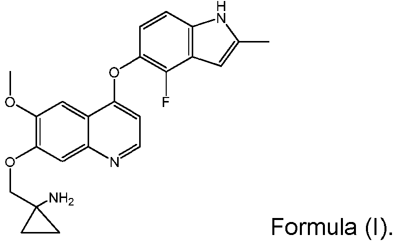

## Patentansprüche

1. Verbindung mit der Formel (I) oder ein pharmazeutisch verträgliches Salz davon zur Verwendung bei der Prävention und/oder Behandlung von Nasopharynxkarzinom, wobei es sich bei dem Nasopharynxkarzinom um fortgeschrittenes und/oder metastasiertes und/oder rezidivierendes Nasopharynxkarzinom handelt,

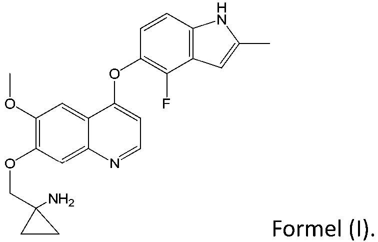

2. Verbindung oder das pharmazeutisch verträgliche Salz davon zur Verwendung nach Anspruch 1, wobei das Nasopharynxkarzinom ein keratinisierendes Plattenepithelkarzinom oder ein nicht-keratinisierendes Nasopharynxkarzinom ist; vorzugsweise ist das nichtkeratinisierende Nasopharynxkarzinom vom differenzierten oder undifferenzierten Typ.

3. Verbindung oder das pharmazeutisch verträgliche Salz davon zur Verwendung nach Anspruch 1, wobei das Nasopharynxkarzinom ein Nasopharynxkarzinom in situ oder ein infiltrierendes Nasopharynxkarzinom ist; vorzugsweise ist das infiltrierende Nasopharynxkarzinom ein Plattenepithelkarzinom, ein Adenokarzinom, ein mikroinvasives Karzinom, ein vesikuläres Kernzellkarzinom oder ein undifferenziertes Nasopharynxkarzinom.

4. Verbindung oder das pharmazeutisch verträgliche Salz davon zur Verwendung nach Anspruch 1, wobei das fortgeschrittene und/oder metastasierte Nasopharynxkarzinom in Halslymphknoten, Milz und/oder Lunge metastasiert.

5. Verbindung oder das pharmazeutisch verträgliche Salz davon zur Verwendung nach Anspruch 1, wobei das Nasopharynxkarzinom nicht chirurgisch entfernt werden kann.

6. Verbindung oder das pharmazeutisch verträgliche Salz davon zur Verwendung nach Anspruch 1, wobei ein Patient mit Nasopharynxkarzinom zuvor eine Chemotherapie, eine monoklonale Antikörperbehandlung und/oder eine Strahlentherapie erhalten hat; vorzugsweise hat der Patient mit Nasopharynxkarzinom nach vorheriger Chemotherapie, monoklonaler Antikörperbehandlung und/oder Strahlentherapie eine Krankheitsprogression.

7. Verbindung oder das pharmazeutisch verträgliche Salz davon zur Verwendung nach Anspruch 6, wobei das zuvor vom Patienten mit Nasopharynxkarzinom erhaltene Chemotherapeutikum Gemcitabin, Capecitabin, Cisplatin, Lobaplatin, Nedaplatin, 5-Fluoruracil, Paclitaxel, Docetaxel und/oder Cyclophosphamid umfasst; wobei das zuvor vom Patienten mit Nasopharynxkarzinom erhaltene monoklonale Antikörper-Therapeutikum Cetuximab, Bevacizumab und/oder SHR-1210 umfasst.

8. Verbindung oder das pharmazeutisch verträgliche Salz davon zur Verwendung nach einem der Ansprüche 1 bis 7, wobei das pharmazeutisch verträgliche Salz der Verbindung mit der Formel (I) ein Salz ist, das durch die Verbindung mit der Formel (I) mit einer der folgenden Säuren gebildet wird: Salzsäure, Bromwasserstoffsäure, Schwefelsäure, Salpetersäure, Phosphorsäure, Essigsäure, Trifluoressigsäure, Propionsäure, Capronsäure, Heptansäure, Cyclopentanpropionsäure, Glykolsäure, Brenztraubensäure, Milchsäure, Malonsäure, Bernsteinsäure, Äpfelsäure, Maleinsäure, Fumarsäure, Weinsäure, Zitronensäure, Benzoesäure, Zimtsäure, Mandelsäure, Methylsulfonsäure, Ethylsulfonsäure, 1,2-Ethandisulfonsäure, 2-Hydroxyethansulfonsäure, Benzolsulfonsäure, p-Chlorbenzolsulfonsäure, p-Toluolsulfonsäure, 3-Phenylpropionsäure, Trimethylessigsäure, t-Butylessigsäure, Dodecylschwefelsäure, Gluconsäure, Glutaminsäure, Hydroxynaphthoesäure, Salicylsäure, Stearinsäure; vorzugsweise ist es ein Hydrochlorid und mehr bevorzugt ein Dihydrochlorid.

9. Verbindung oder das pharmazeutisch verträgliche Salz davon zur Verwendung nach einem der Ansprüche 1 bis 7, wobei eine tägliche Verabreichungsdosis 2 mg bis 20 mg, vorzugsweise 5 mg bis 20 mg, mehr bevorzugt 10 mg bis 16 mg, mehr bevorzugt 10 mg bis 14 mg, mehr bevorzugt 8 mg, 10 mg, 12 mg, 14 mg oder 16 mg beträgt.

10. Verbindung oder das pharmazeutisch verträgliche Salz davon zur Verwendung nach einem der Ansprüche 1 bis 7, wobei die Verbindung mit der Formel (I) oder das pharmazeutisch verträgliche Salz davon durch ein Intervall-Verabreichungsschema verabreicht wird, das Verabreichungszeiträume und Ruhezeiträume umfasst, und wobei ein Verhältnis des Verabreichungszeitraums zum Ruhezeitraum in Tagen 2 : 0,5-5, vorzugsweise 2 : 0,5-3, mehr bevorzugt 2 : 0,5-2 und mehr bevorzugt 2 : 0,5-1 beträgt.

11. Verbindung oder das pharmazeutisch verträgliche Salz davon zur Verwendung nach Anspruch 10, wobei das Intervall-Verabreichungsschema, das Verabreichungszeiträume und Ruhezeiträume umfasst, eine kontinuierliche Verabreichung über 2 Wochen und eine Ruhephase von 2 Wochen oder eine kontinuierliche Verabreichung über 2 Wochen und eine Ruhephase von 1 Woche oder eine kontinuierliche Verabreichung über 5 Tage und eine Ruhephase von 2 Tagen ist.

12. Pharmazeutische Zusammensetzung zur Verwendung bei der Behandlung von Nasopharynxkarzinom, umfassend eine Verbindung mit der Formel (I) oder ein pharmazeutisch verträgliches Salz davon und mindestens einen pharmazeutisch verträglichen Träger.

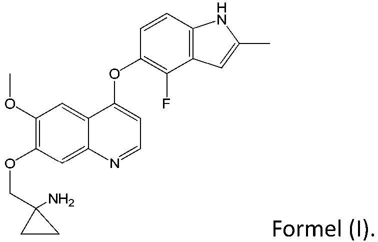

13. Kit zur Verwendung bei der Behandlung von Nasopharynxkarzinom, umfassend (a) mindestens eine Einheitsdosis einer pharmazeutischen Zusammensetzung einer Verbindung mit der Formel (I) oder eines pharmazeutisch verträglichen Salzes davon und (b) Anweisungen zur Behandlung von Nasopharynxkarzinom,

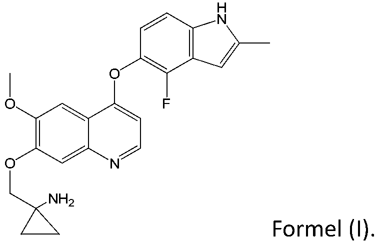

## Revendications

1. Composé de formule (I) ou sel pharmaceutiquement acceptable de celui-ci, pour utilisation dans la prévention et/ou le traitement du carcinome du nasopharynx, dans lequel le carcinome du nasopharynx est un carcinome du nasopharynx avancé et/ou métastasique et/ou récurrent,

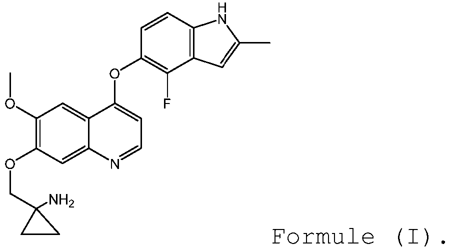

2. Composé ou sel pharmaceutiquement acceptable de celui-ci pour utilisation selon la revendication 1, le carcinome du nasopharynx est un carcinome épidermoïde kératinisant ou non kératinisant ; de préférence, le carcinome du nasopharynx non kératinisant est de type différencié ou indifférencié.

3. Composé ou sel pharmaceutiquement acceptable de celui-ci pour utilisation selon la revendication 1, dans lequel le carcinome du nasopharynx est un carcinome du nasopharynx *in situ* ou infiltrant ; de préférence, le carcinome du nasopharynx infiltrant est un carcinome épidermoïde, un adénocarcinome, un carcinome microinvasif, un carcinome à noyau vésiculaire ou un carcinome du nasopharynx indifférencié.

4. Composé ou sel pharmaceutiquement acceptable de celui-ci pour utilisation selon la revendication 1, dans lequel le carcinome du nasopharynx avancé et/ou métastatique métastase à la lymphe du cou, la rate et/ou le poumon.

5. Composé ou sel pharmaceutiquement acceptable de celui-ci pour utilisation selon la revendication 1, dans lequel le carcinome du nasopharynx ne peut pas être éliminé chirurgicalement.

6. Composé ou sel pharmaceutiquement acceptable de celui-ci pour utilisation selon la revendication 1, dans lequel un patient atteint d'un carcinome du nasopharynx a reçu préalablement une chimiothérapie, un traitement par anticorps monoclonaux et/ou une radiothérapie ; de préférence, le patient atteint d'un carcinome du nasopharynx a une progression de la maladie après avoir reçu préalablement une chimiothérapie, un traitement par anticorps monoclonaux et/ou une radiothérapie.

7. Composé ou sel pharmaceutiquement acceptable de celui-ci pour utilisation selon la revendication 6, dans lequel l'agent chimiothérapeutique reçu précédemment par le patient atteint d'un carcinome du nasopharynx comprend la gemcitabine, la capécitabine, le cisplatine, la lobaplatine, la nédaplatine, le 5-fluorouracile, le paclitaxel, le docétaxel et/ou le cyclophosphamide ; l'agent thérapeutique d'anticorps monoclonaux précédemment reçu par le patient atteint d'un carcinome du nasopharynx comprend le cétuximab, le bévacizumab et/ou SHR-1210.

8. Composé ou sel pharmaceutiquement acceptable de celui-ci pour utilisation selon l'une quelconque des revendications 1 à 7, dans lequel le sel pharmaceutiquement acceptable du composé de formule (I) est un sel formé par le composé de formule (I) avec l'un quelconque des acides suivants : acide chlorhydrique, acide bromhydrique, acide sulfurique, acide nitrique, acide phosphorique, acide acétique, acide trifluoroacétique, acide propionique, acide caproïque, acide heptanoïque, acide cyclopentanepropionique, acide glycolique, acide pyruvique, acide lactique, acide malonique, acide succinique, acide malique, acide maléique, acide fumarique, acide tartrique, acide citrique, acide benzoïque, acide cinnamique, acide mandélique, acide méthylsulfonique, acide éthylsulfonique, acide 1,2-éthanedisulfonique, acide 2-hydroxyéthanesulfonique, acide benzènesulfonique, acide p-chlorobenzènesulfonique, acide p-toluènesulfonique, acide 3-phénylpropionique, acide triméthylacétique, acide t-butylacétique, acide dodécylsulfurique, acide gluconique, acide glutamique, acide hydroxynaphtoïque, acide salicylique, acide stéarique ; de préférence est un chlorhydrate, et préférablement un dichlorhydrate.

9. Composé ou sel pharmaceutiquement acceptable de celui-ci pour utilisation selon l'une quelconque des revendications 1 à 7, dans lequel une dose d'administration quotidienne est de 2 mg à 20 mg, de préférence de 5 mg à 20 mg, plus préférablement de 10 mg à 16 mg, plus préférablement de 10 mg à 14 mg, plus préférablement de 8 mg, 10 mg, 12 mg, 14 mg ou 16 mg.

10. Composé ou sel pharmaceutiquement acceptable de celui-ci pour utilisation selon l'une quelconque des revendications 1 à 7, dans lequel le composé de formule (I) ou le sel pharmaceutiquement acceptable de celui-ci est administré par un régime d'administration à intervalle comprenant des périodes d'administration et des périodes de repos, et dans lequel un rapport de la période d'administration à la période de repos en jours est de 2 : 0,5 de préférence 2 : 0,5-3, plus préférablement 2 : 0,5-2, et plus préférablement 2 : 0,5-1.

11. Composé ou sel pharmaceutiquement acceptable de celui-ci pour utilisation selon la revendication 10, dans lequel le régime d'administration à intervalle comprenant des périodes d'administration et des périodes de repos est administré en continu pendant 2 semaines et au repos pendant 2 semaines, ou administré en continu pendant 2 semaines et au repos pendant 1 semaine, ou administré en continu pendant 5 jours et au repos pendant 2 jours.

12. Composition pharmaceutique pour utilisation dans le traitement d'un carcinome du nasopharynx, comprenant un composé de formule (I) ou un sel pharmaceutiquement acceptable de celui-ci, et au moins un support pharmaceutiquement acceptable

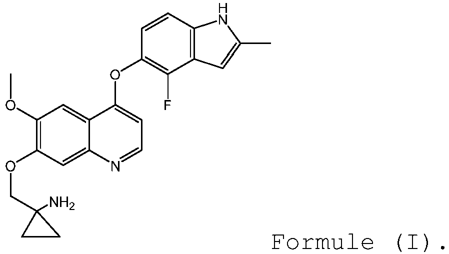

13. Kit pour utilisation dans le traitement du carcinome du nasopharynx, comprenant (a) au moins une dose unitaire d'une composition pharmaceutique d'un composé de formule (I) ou d'un sel pharmaceutiquement acceptable de celui-ci, et (b) des instructions pour un traitement d'un carcinome du nasopharynx,
